# EUROPEAN PATENT SPECIFICATION

(11) **EP 0 786 233 B1**
(45) Date of publication and mention of the grant of the patent: **31.10.2001**
(21) Application number: 97100651.5
(22) Date of filing: 17.01.1997
(51) Int. Cl.: A61B 10/00

(54) **Biopsy surgical appliance**
Chirurgisches Gerät für Biopsie
Appareil chirurgical pour biopsie

(30) Priority: 26.01.1996 IT BO960033
(43) Date of publication of application: 30.07.1997
(73) Proprietor: Allegiance Healthcare Corporation, McGaw Park, IL 60085 (US)
(72) Inventor: Bauer, Alberto, Santo Domingo (DO)
(74) Representative: Gustorf, Gerhard, Dipl.-Ing.

(56) References cited:
- EP-A- 0 536 888
- WO-A-91/01112
- US-A- 4 924 878
- US-A- 4 958 625

## Description

The present invention relates to a biopsy surgical appliance as set forth in the preamble of claim 1.
- Biopsy surgical appliances are commonly used to remove a sample of body tissue in order to be able to examine it while causing the least possible damage to the patient subject to removal.
- Several biopsy surgical appliances are currently known. They substantially include a needle made up of a thin probe ending with a stylet arranged coaxially inside a hollow circular element acting as cannula, and a control device for needle operation.
- With reference to patent US-4.958.625, current appliances include two sliders, arranged aligned on the same needle axis, elastically loadable through a respective spring, where a first slider carries the stylet and the other slider carries the cannula.
- Such sliders are associated with hooking/releasing holding devices capable of keeping the same sliders in the wound or axial load position in order to be able to carry out, through controls, two different executions of the removal: a first execution where said stylet slider and said cannula slider are released in quick sequence by a single control respectively; and a second execution where said stylet slider is released by a first control and said cannula slider is released by a second control.
- More particularly, the above-mentioned patent envisages that hooking/releasing holding means of cannula slider are associated and/or co-operating with the stylet slider, where releasing of said cannula slider holding means occurs and may occur when the stylet slider has reached the end of its advancement stroke only.
- Said acknowledged technique presents some substantial inconveniences which vary according to the type of removal to be carried out mentioned hereinafter.
- With reference to the first type of removal, that is the one including stylet and cannula advanced in quick sequence, sometimes, in case of a particularly resistant tissue, there may be the inconvenience that execution of the removal is not performed correctly since the stylet slider, not reaching advancement limit stop, does not exert the pressure on cannula slider holding means necessary for their release.
- This particular malfunction may cause even serious damage to patient since it is necessary to move needle with the stylet partially fed and the cannula backed, that is moving needle before cannula has made the cut of the sample inside the pierced tissue.
- With reference to second type of removal, that is the one comprising a first stylet advancement by a first control and then a second cannula advancement by a second control, sometimes, in the afore-mentioned case of particularly resistant tissue, there may be the inconvenience that the operator shoots stylet at first and then, when he presses the push-button for cannula, the release does not occur since the stylet slider, as mentioned above, is not in limit stop position.

A biopsy surgical appliance as described in the preamble of claim 1 is known from US-A 4 924 878. Here, the holding/releasing means have only one control push-button.

It is an object of the present invention to solve the above mentioned inconveniences and to provide a biopsy appliance that may be used in two different types of removal of the tissue to be chosen by the surgeon.

According to the invention, this object is accomplished by the characterising features of claim 1. Further useful characteristics are exposed in the dependent claims.

The use of this type of biopsy surgical appliance has the advantage that the release of the slider carrying cannula occurs also in case the slider carrying stylet does not reach the limit stop. Further advantages of this invention essentially consist of the fact that one always succeeds in performing a correct removal of the sample, of the fact that by adopting the removal method in quick sequence cannula always performs the cutting stroke and of the fact that by adopting the two-phase removal method cannula always performs the cutting stroke.
- Further characteristics and advantages of this invention will be particularly evident from the following detailed description of one of its preferred form of practical accomplishment herewith given as a non-restrictive example made with reference to pictures in the attached drawings where:
   - Picture 1 is a prospective view illustrating the biopsy surgical appliance object of this invention in which the holding box upper part has been lifted;
   - Picture 1A is an exploded prospective view illustrating components of biopsy surgical appliance of picture 1;
   - Pictures 2 and 2A are a bottom view and a sectional view respectively along line A-A of picture 2 of a component of pictures 1 and 1A;
   - Pictures 3 and 3A are a plan view and a sectional view respectively along line A-A of picture 3 of a component of pictures 1 and 1A;
   - Pictures 4 and 4A are a side view and a sectional view respectively along line A-A of picture 4 of a component of pictures 1 and 1A;
   - Pictures 5 and 5A are a plan view and a sectional view respectively along line A-A of picture 5 of a component of pictures 1 and 1A;
   - Pictures 6 and 6A are a side view and a sectional view respectively along line A-A of picture 6 of a component of pictures 1 and 1A;
   - Picture 7A is a top schematic view in transparency through upper wall of the appliance case of picture 1 illustrating the appliance in an idle state and/or in a first position of the load cycle;
   - Picture 7B is a sectional top view along the median horizontal plane of the surgical appliance in the state of Pict. 7A;
   - Picture 7C is a sectional view along line C-C of picture 7A,
   - Picture 7D is a sectional view along line D-D of picture 7A;
   - Picture 8B is a view illustrating an operative detail;
   - Picture 9A is a top schematic view in transparency through upper wall of appliance case of picture 1 illustrating the appliance in a second position of the load cycle;
   - Picture 9B is a sectional top view along the horizontal median plane of surgical appliance in the position of Pict. 9A;
   - Picture 9C is a sectional view along line C-C of picture 9A,
   - Picture 9E is a sectional view along line E-E of picture 9C;
   - Picture 10A is a top schematic view in transparency through upper wall of appliance case of picture 1 illustrating the appliance in a third position of load cycle;
   - Picture 10B is a sectional view along median horizontal plane and from surgical appliance top in the position of Pict. 9A;
   - Picture 11A is a schematic top view in transparency through upper wall of appliance case of picture 1 illustrating the appliance in a third position of the load cycle;
   - Picture 11B is a sectional view along median horizontal plane and from the surgical appliance top in the position of Pict. 11A;
   - Picture 11D is a sectional view along line D-D of picture 11A;
   - Picture 11E is a sectional view along line E-E of picture 11D;
   - Picture 12A is a schematic top view in transparency of the upper wall of appliance case of picture 1 illustrating the appliance in a forth and last position of load cycle;
   - Picture 12B is a sectional view along horizontal median plane and from surgical appliance top in the position of Pict. 12A;
   - Picture 12D is a sectional view along plane D-D of picture 12A;
   - Picture 12E is a sectional view along line E-E of picture 12D;
   - Pictures 13D and 14D are two schematic longitudinal sectional views illustrating a first and a second operative phase in the execution of a first type removal aiming at advancing stylet and cannula in quick sequence.
   - Pictures 15D and 16D are two sectional longitudinal schematic views illustrating a first operative phase and a second operative phase in the execution of a removal of the type aiming at advancing the stylet by a first control and the cannula by a second control.
   - With reference to picture 1 and 1A, the illustrative appliance extends lengthwise along a y-axis and outside it is made up of a case shaped in two semi-shells 1a and 1b.
   - Upper Shell 1a, see also pict. 2 and 2A, is made up of a front wall 2, a side wall 3, a back wall 4, a side wall 5 and an upper wall 6, where front wall 2 includes a semi-hole 7a for the passage of a biopsy needle 8. -Upper wall 6 includes a slot 9, a first portion 10 bending in a transversal plane and a second portion 11 bending in a longitudinal plane. -Inner surface of wall 6 includes: a first longitudinal elevation 12 which configures a longitudinal sliding channel 12a with wall 5; a couple of elevations 13 and 14 which configure a central longitudinal sliding channel 13a between them; a second elevation 15 which configures a longitudinal sliding channel 15a with wall 3; a holding tooth 16; a pushing tooth 17; a holding tooth 18, a pushing tooth 19 and a pushing tooth 10a; where said pushing teeth 17 and 19 are carried by bending part 11, and said tooth 10a is carried by bending part 10, so as to be mobile downwards if a strength is applied inwards on outer bending part 11 or 10.
   - Lower Shell 1b, see also pict. 3 and pict. 3A, includes: front wall 21, a side wall 22, a back wall 23, a side wall 24 and a lower wall 25, where front wall 21 presents a half hole 7b for the passage of needle 8 mentioned above. -Lower wall 25 includes a longitudinal elevation 26 which configures a longitudinal sliding channel 26a with wall 24, a double series 27a, 27b, 27c and 28a, 28b, of elevations aligned lengthwise which configure a central sliding channel 271 between them, an elevation 29 which configures a longitudinal sliding channel 29a with wall 22 and, in the centre, a passing slot 30.
   - Inside shells 1a and 1b, see in particular Pict. 1 and 1A, three elements sliding lengthwise, a cannula slider 31, a loader slider 32 and a stylet slider 33 are placed.
   - With reference to said cannula slider 31, see also Pict. 4, 4A, 2A, it presents, for its guided longitudinal sliding, a couple of upper flaps 34 and 35 suitable for sliding in the longitudinal channel 12a of upper shell 1a and, below, a couple of flaps 36 and 37 suitable for sliding in the longitudinal channel 26a of lower shell 1b. -The four mentioned flaps 34, 35, 36, 37 are carried by a main body 40 presenting on its top an elastic bridge 41 equipped with a holding mobile tooth 42 and inside said body 40 a back housing 43 and an additional front housing 44 are provided. -Housing 43 carries a spring 45 which has its opposite end triggerd on a pin 46 equipped with a longitudinal hole 52 where said pin 46 is carried by wall 23 of shell 1b and by two shoulders 72 and 73. -Housing 44 is suitable for housing a spring 70 and spring guide rod 49 with a diameter which is smaller than that of said hole 52 so as to pass through the interspace between said two housings 43 and 44 for the reasons described and explained hereinafter. -Front part of said cannula slider 31 includes a head 38 extending transversally beyond y-axis having coaxially with respect to the latter a hole 39 suitable for carrying cannula 80 of needle 8.
   - With reference to said loader slider 32, see also Pict. 5 and 5A, it slides lengthwise guided in a upper sliding channel 13a of shell 1a and in lower guiding channel 271 of shell 1b. -Such a loader slider 32 presents a fork central body with tines 47a and 47b, a head 48, spring guiding rod 49 on which spring 70 is trigged, and a couple of wings 50a and 50b suitable for sliding inside respective slots 9 and 30 of shells 1a and 1b.
   - With reference to head 48, it presents a "U" reversed configuration in which two transversal guides 51a and 51b are made and suitable for carrying a square element 53 sliding transversally and loaded elastically by a spring 55, which, see also Pict. 7B, stops on a limit stop 69 to inhibit its exit. -Said square 53, when inserted into guides 51a and 51b, presents its two vertical sides 53s and 53d mobile transversally and respectively destined to selectively meet front faces of cannula slider 32 and stylet slider 33 as better described hereinafter.
   - With reference to said stylet slider 33, see also Pict. 6 and 6A, it presents for its guided longitudinal sliding a couple of upper flaps 56 and 57 suitable for sliding in the longitudinal channel 15a and, below, a couple of flaps 58 and 59 suitable for sliding in the lower longitudinal channel 29a. -The mentioned flaps are carried by a main body 60 which presents on its top an elastic bridge 61 equipped with a mobile tooth 62 and a housing 63 inside. -Housing 63 carries a spring 64 which has its opposed end trigged on a pin 65 carried by wall 23 of lower shell 1b. -Back part of said stylet slider 33 includes a head 66, extending transversally beyond y-axis, which includes a hole 67 that is coaxial with respect to said y-axis in which a stylet back and/or control end 68 of needle 8 is trigged.
- With reference to pictures 7A, 7B, 7C and 7D, they illustrate the appliance at the beginning of its loading for the subsequent removal, that is in a first idle position.
- In said idle position sliders 31, 32 and 33 are pressed on front walls 2 and 21 by the respective springs 45, 70 and 64.
- Square 53, sliding transversally, is loaded by spring 55 which presses it on limit stop 69 and, see Pict. 7B, it presents two mobile strikers 53s and 53d, of which striker 53s is aligned lengthwise to meet inclined plane 31a of element 31 and striker 53d is aligned lengthwise to meet element 33 front part.
- To obtain appliance loading in view of the removal the operator must wind cannula slider 31 first and then stylet slider 33.
- Such operator acts to move the loader slider 32 backwards by grasping the two wings 50a and 50b extending outside shells 1a and 1b.
- With reference to picture 8B, which illustrates a special operative arrangement, when the loader slider 32 starts moving towards appliance back part to begin winding or loading operations, striker 53s interferes with inclined plane 31a a thus entailing a displacement of the relative square 53 on the left, so as striker 53d does not interfere with element 33 front part.
- With reference to pictures 9A, 9B and 9C, loader slider 32 has been pulled backwards and it has dragged cannula slider 31 with itself. -Said cannula slider, moving backwards, has compressed spring 45 and mobile holding tooth 42, carried by elastic bridge 41, has hooked fixed holding tooth 16 carried by shell 1a. -With reference to this hooking, see pictures 9C and 9E, please note that mobile holding tooth 42 clutches with fixed tooth 16 and that bridge 41 includes an upper portion 41a, extending lengthwise and arranged transversally at the tooth side 42, which is aligned vertically with pushing tooth 17 carried by bending part 11, so that, by pressing said bending part 11, pushing tooth 17 presses on bridge 41 and as it is pressed down it is lowered with subsequent release of teeth 16 and 42 for the reasons described hereinafter.
- With reference to pictures 10A and 10B, loader slider 32 after loading cannula slider 31 as mentioned above, returns to the former position by pushing spring 70 while square 53 loaded by spring 55 positions on limit stop 69.
- With reference to pictures 11A, 11B and 11D, loader slider 32 has been pulled again towards the back and has dragged stylet slider 33 with itself. -In this respect please note that since cannula slider 31 is positioned towards the back, square 53 is not subject to transversal displacements by inclined plane 31a.
- Stylet slider 33, moving backwards, has compressed spring 64 and mobile holding tooth 62, carried by elastic bridge 61, has hooked fixed holding tooth 18 carried by shell 1a. -With reference to that hooking, see Pict. 11E, please note that mobile holding tooth 62 clutches with fixed tooth 18 and that bridge 61 includes an upper portion 61 a, extending lengthwise and arranged transversally at tooth side 62, which is aligned vertically with pushing tooth 19 carried by bending part 11, so that, by pressing said bending part 11, presses on tooth 19 pushes bridge 61 and as it is pressed down it is lowered with subsequent release of teeth 62 and 18 for the reasons described hereinafter.
- With reference to pictures 12A and 12B, loader slider 32 after loading stylet slider 33 as mentioned above, returns to its former position by pushing spring 70.
- After executing the above-mentioned winding operations, see Pict. 12D and 12E, stylet slider 33 is wound and held in position through hooking of teeth 18-62 while cannula slider 31 is wound and held in position through hooking 42-16. -Furthermore, bending part 11 presents two pushing teeth 19 and 17 where pushing tooth 19 can press elastic bridge 61 of cannula slider 33 in order to release it and pushing tooth 17 can press elastic bridge 41 of stylet slider 31 to release it.
- With reference to the type of removal to be performed, the operator must wind the appliance as described above first and then insert needle 8 in the tissue and position its free point in the area destined to removal.
- At this point the operator may opt for two different types of removal, a first type where the stylet 68 and the cannula 80 are advanced in successive quick sequence by a single control, or a second type of removal where stylet 68 is advanced by a first control and cannula 80 by a second control.
- With reference to first type of removal, see Pict. 13D and 14D the operator, applying a strength directed from outside to inside on bending element 11, moves pushing teeth 19 and 17 downwards with different movements, where tooth 19 during bending phase executes downwards movements which are greater with respect to tooth 17.
- More particularly, flexure of bending element 11 entails first stylet slider 33 release through pushing tooth 19 pressure on bridge 61 which involves mobile holding tooth 62 to be released from fixed holding tooth 18 with subsequent advancement of stylet slider 33 by pre-wound spring 64, see Pict. 13D. -Then, due to further flexure of bending element 11, it entails mobile holding tooth 42 release from fixed holding tooth 16 with subsequent advancement of cannula slider 31 by pre-wound spring 45, see Pict. 14D.
- In such a manner advancement of stylet 68 and cannula 80 is obtained in quick sequence as required for first type of removal, and in case stylet 68 and therefore the relative slider 33 because of a particularly hard tissue do not reach advancement limit stop, cannula 80 executes its cutting stroke all the same since the release of the holding means 42-16 is independent from the system relative to stylet slider 33.
- With reference to second type of removal, see Pict. 15D and 16D, the operator, applying a first control through a first strength FA directed from outside to inside on the bending element 10, moves bridge 61 downwards through pushing tooth 10a, thus entailing the release of holding tooth 62 from holding tooth 18 with subsequent advancement of stylet slider 33 by pre-wound spring 64, see Pict. 15D. -Then, after examining the correct arrangement of needle point 8 by magnetic resonance, computed axial tomography or other systems, the second control is operated through the application of a strength FB on bending element 11 by releasing cannula slider 31 as aforementioned.
- In such a manner a advancement of cannula 80 is obtained also in case slider 33, because of a particularly hard tissue found stylet point 68, does not reach advancement limit stop, since the release of the holding means 42-16 of cannula slider 31 are not associated with stylet slider 33.
- The description of the above mentioned appliance is given as a non-restrictive example and thus it is evident that any modification or variation may be introduced into it according to practise and its use or employment and however within the scope of the following claims.

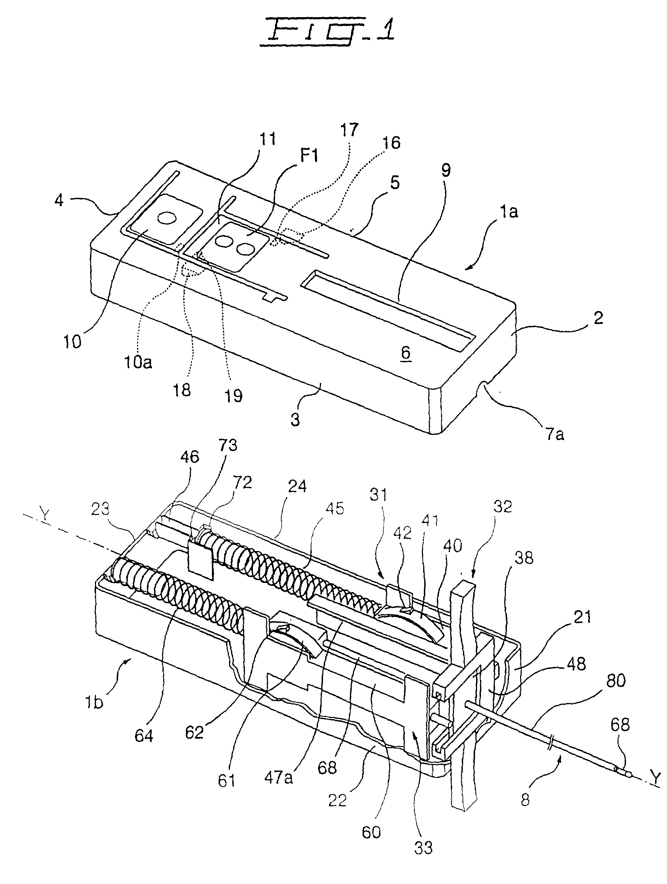

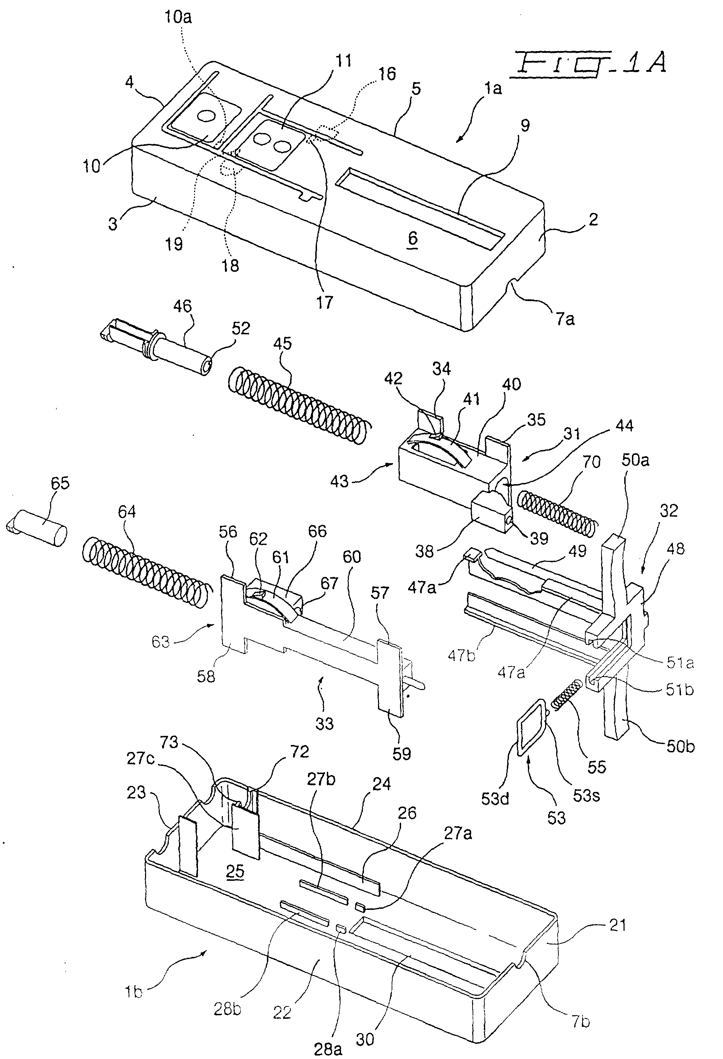

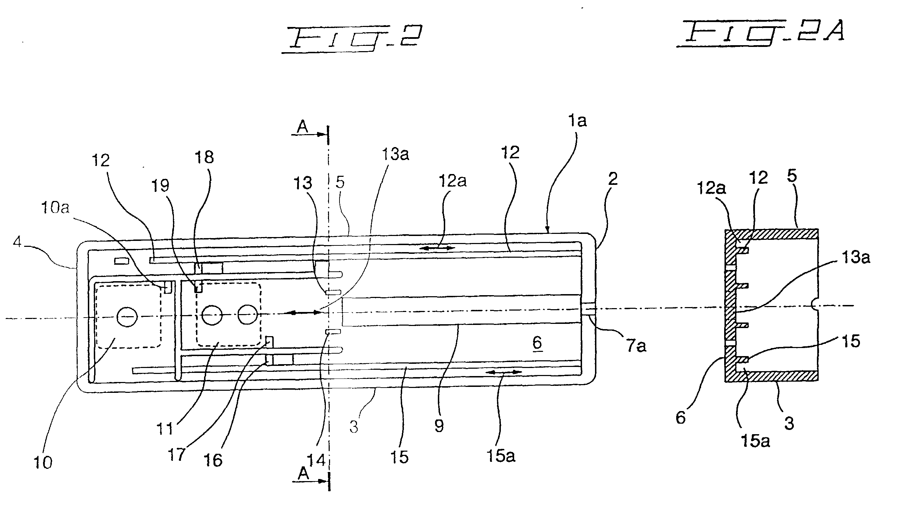

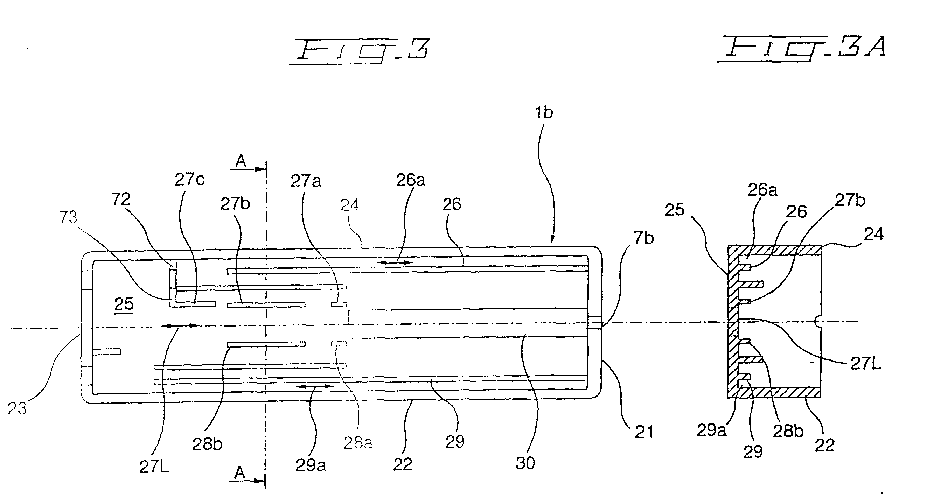

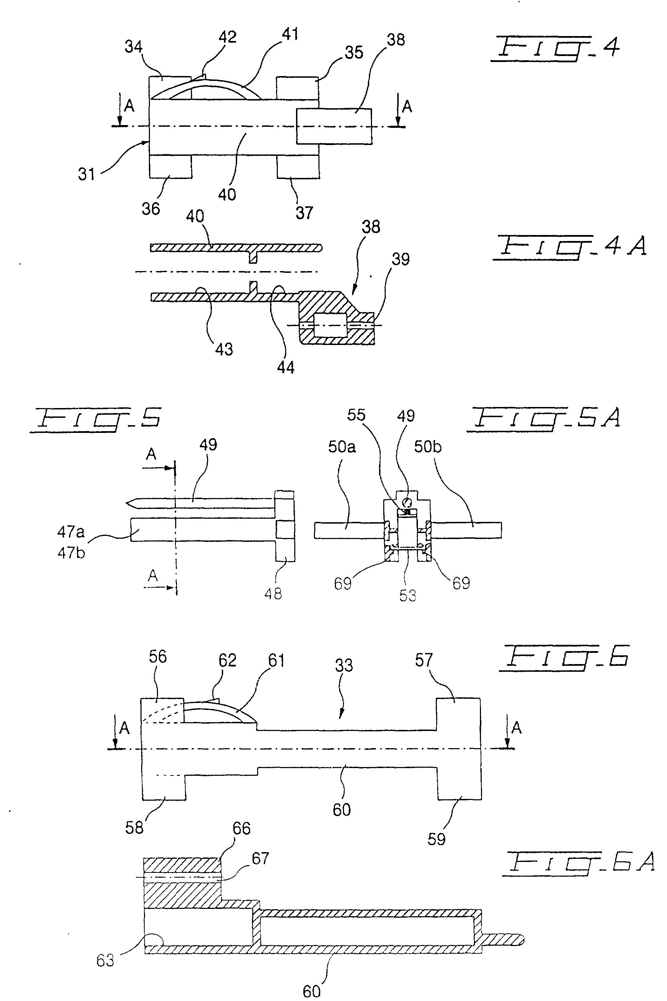

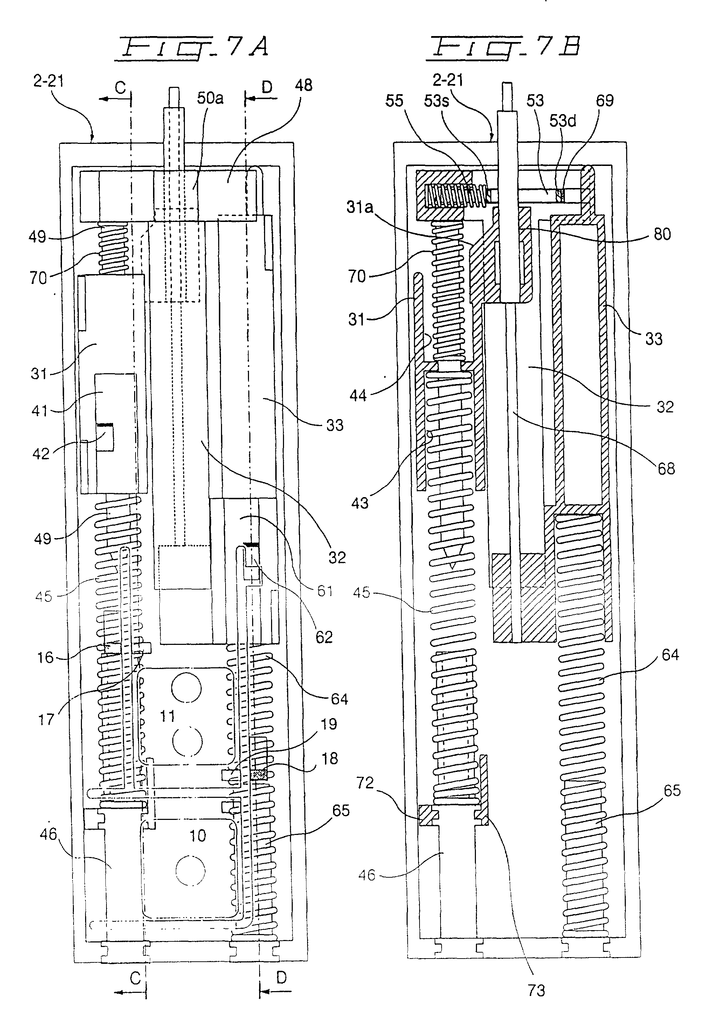

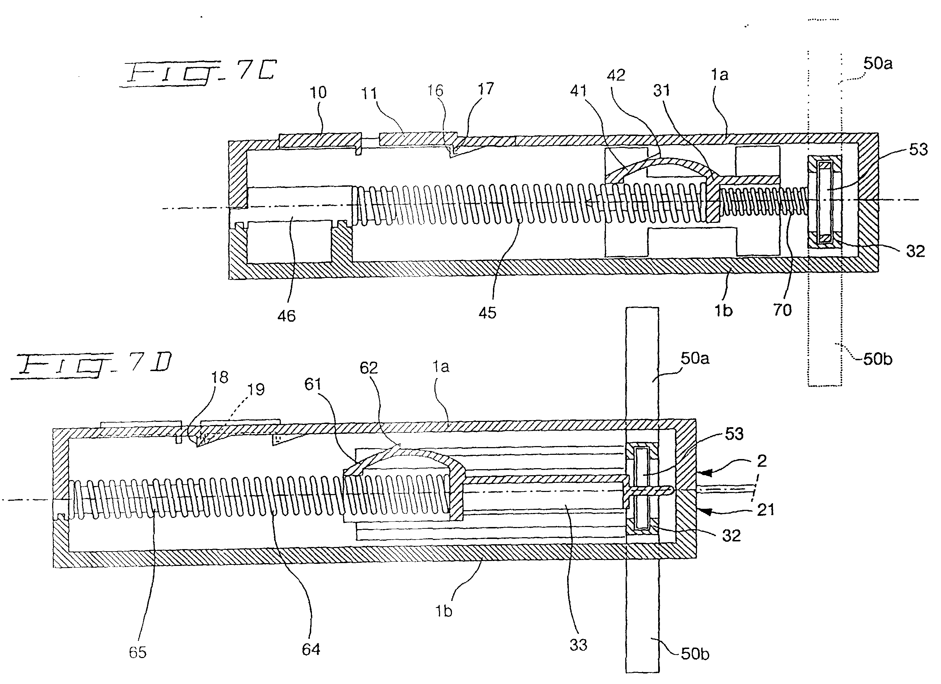

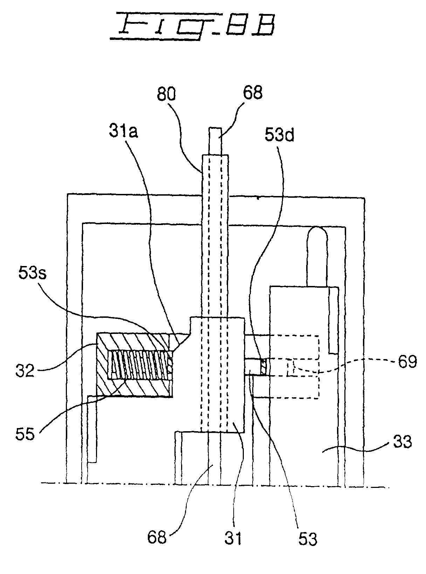

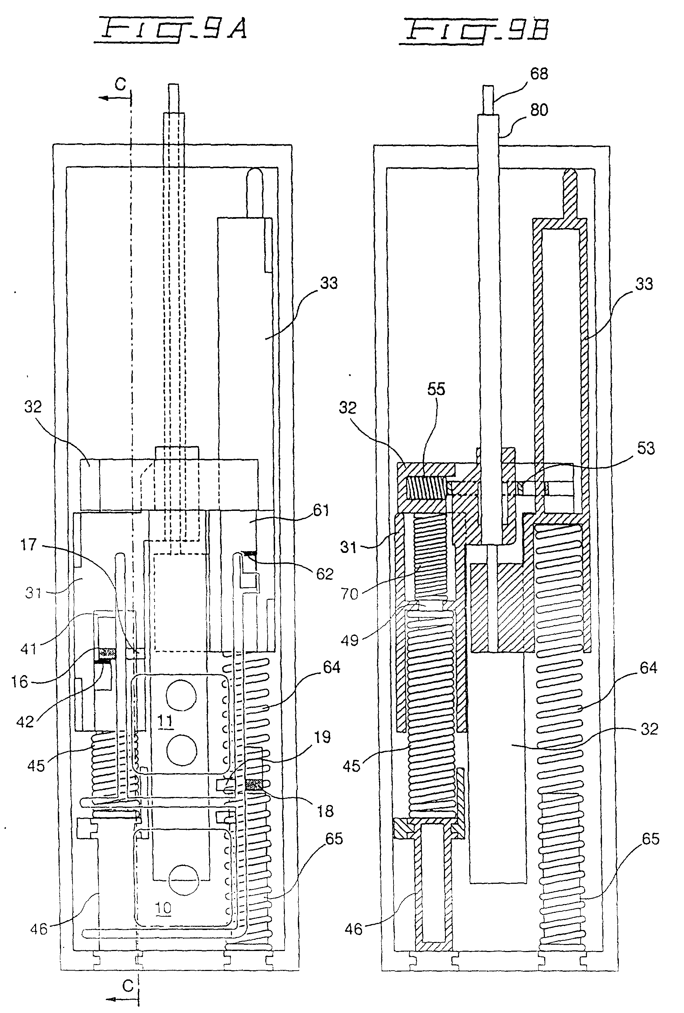

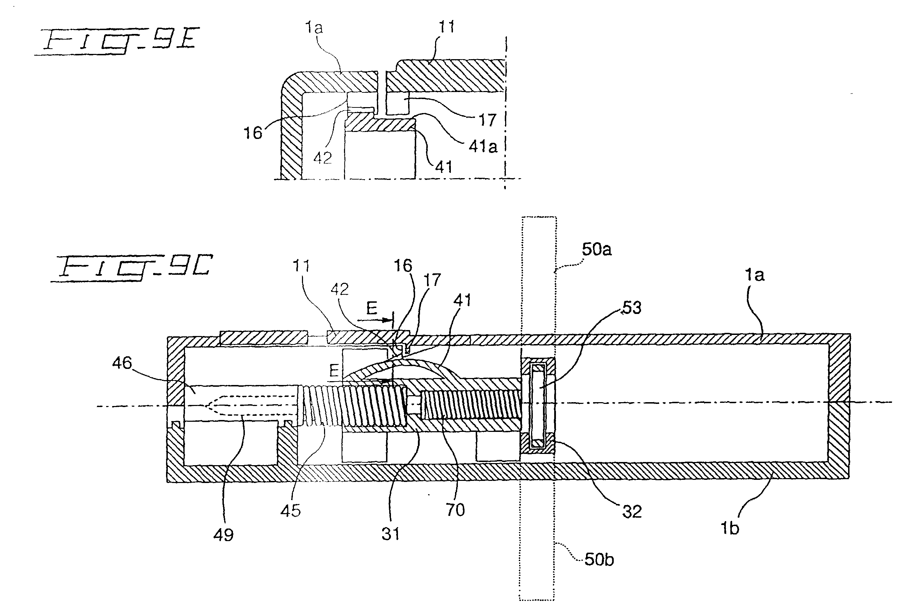

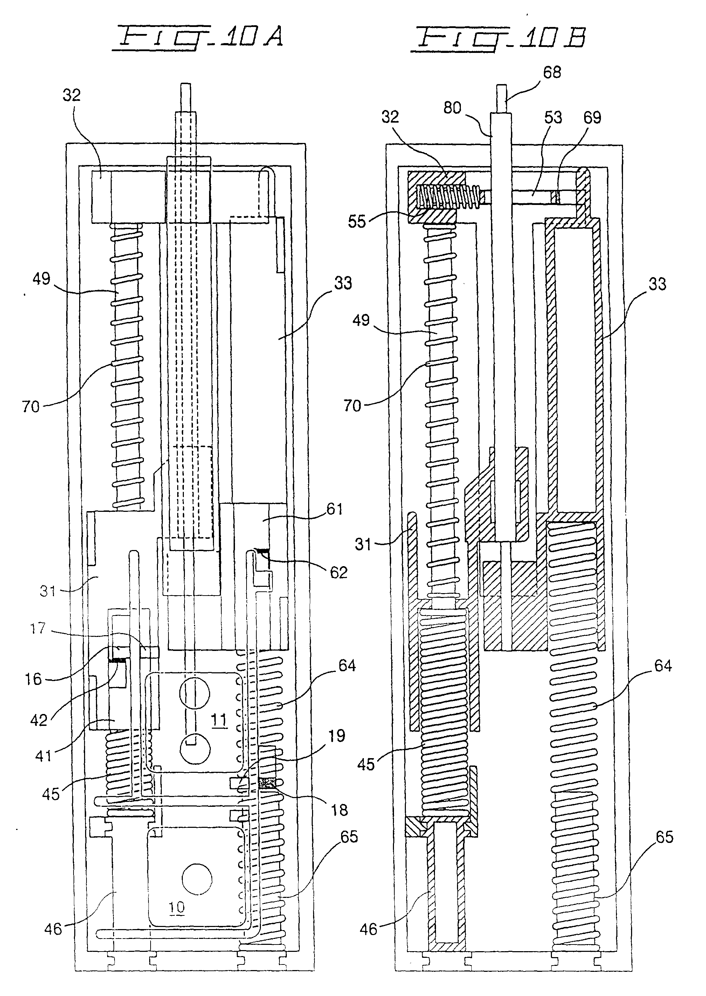

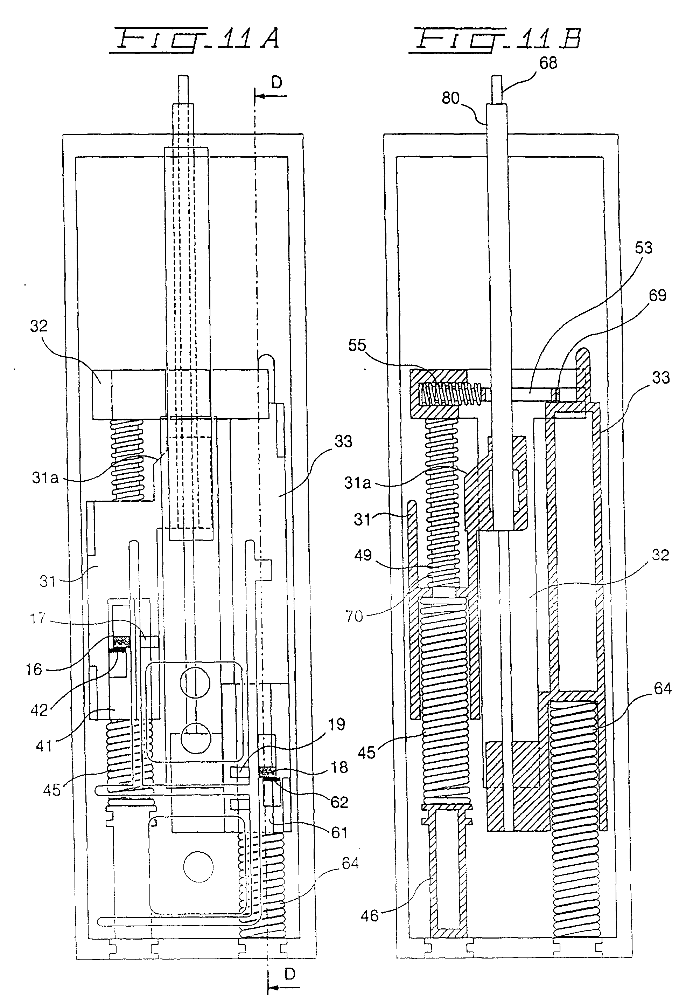

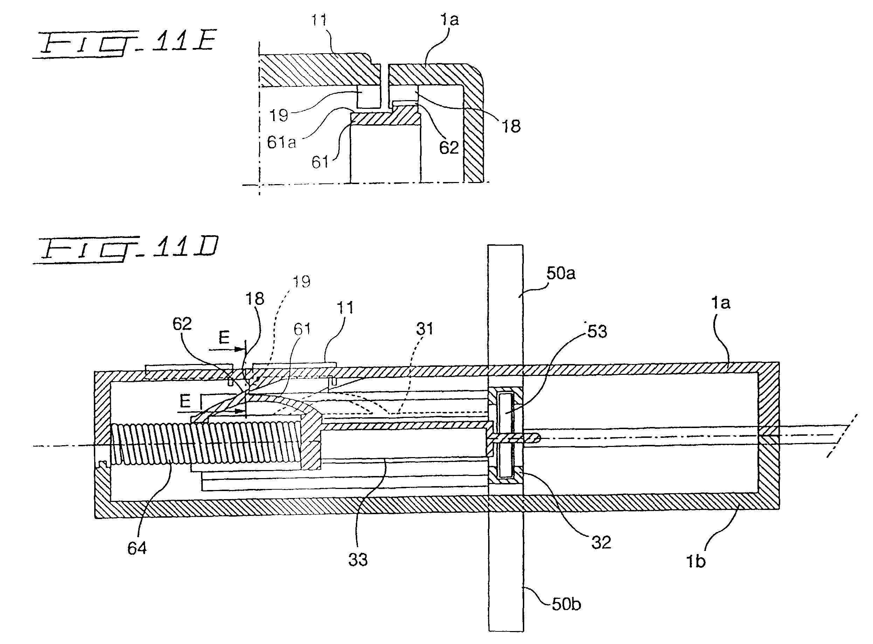

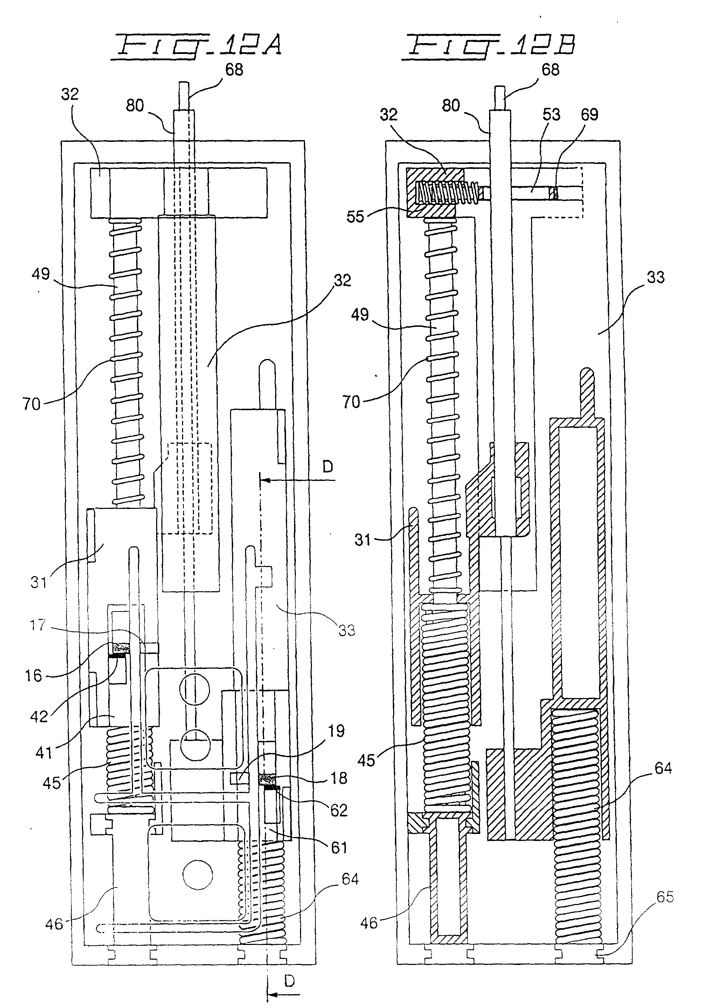

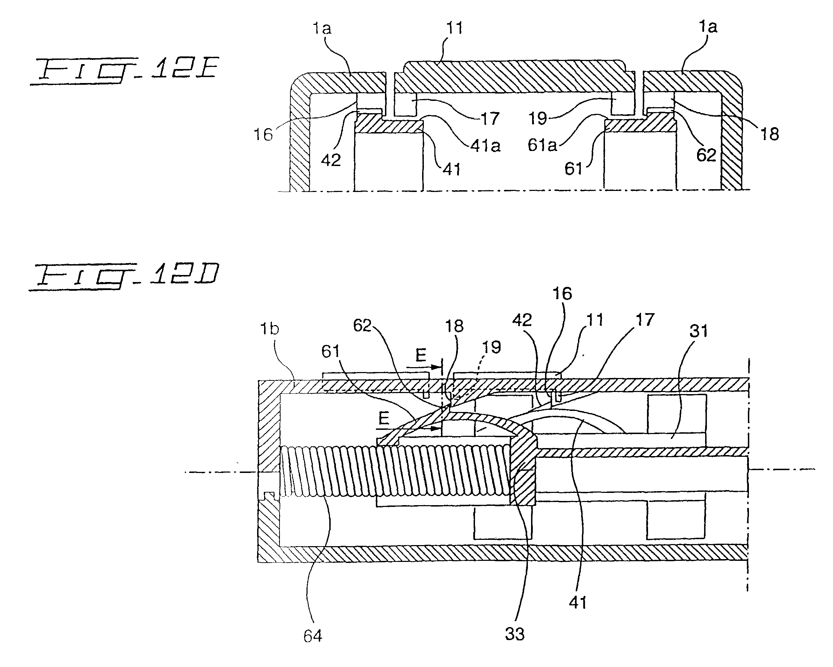

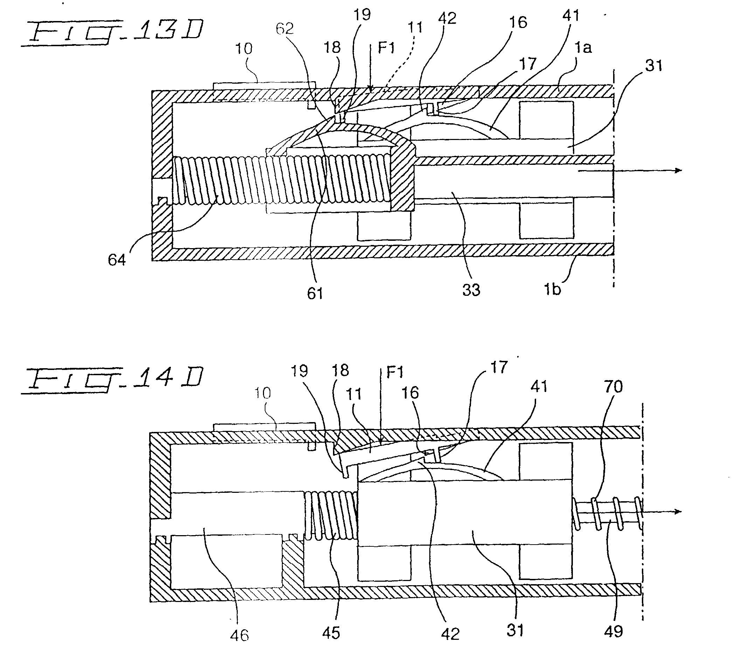

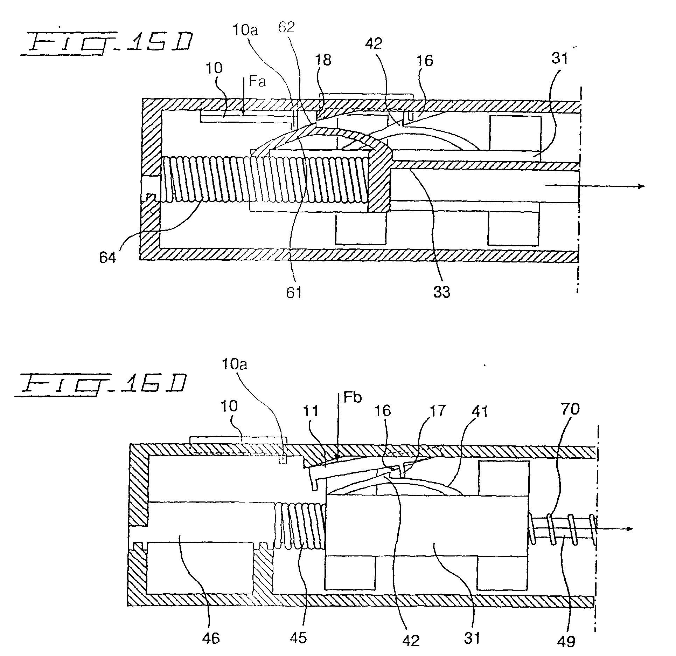

## Claims

1. Biopsy surgical appliance including a needle (8) made up of a stylet (68) and a cannula (80) arranged coaxially and extending axially along a longitudinal axis (y), having a free end to be inserted into the removal area and the other end placed in a box-handpiece (1) containing control means to automatize removal operations, said means comprising:
- a stylet slider (33) slidingly guided lengthwise inside said handpiece (1) suitable for carrying and advancing stylet (68) lengthwise;
- a cannula slider (31) slidingly guided lengthwise inside said handpiece (1) suitable for supporting and advancing cannula (80) lengthwise;
- first elastic means (64) suitable for elastically loading said stylet slider (33) lengthwise;
- second elastic means (45) suitable for elastically loading said cannula slider (31) lengthwise;
- holding/releasing means to hold said stylet slider (33) and said cannula slider (31) in the loaded position and to release them in automatic respective quick sequence or in separate sequence;
- means to carry said stylet slider (33) and said cannula slider (31) in the loaded position;
- said stylet slider (33) sliding in a longitudinal plane arranged laterally with respect to axis (y) of needle (8) and including a head (66) extending towards and beyond said axis (y) and suitable for bringing stylet (68) coaxially with respect to said axis (y) ;
- said cannula slider (31) sliding in a longitudinal plane arranged laterally with respect to axis (y) of needle (8) and not interfering in the operative range of stylet slider (33) and including a head (38) extending towards and beyond said axis (y) and suitable for bringing cannula (80) coaxially with respect to said axis (y);
- **characterised by** the fact that said holding/releasing means include a first independent hooking/releasing device (18, 62) for stylet slider (33), a second independent hooking/releasing device (16, 42) for cannula slider (31), and two independent separate control push-buttons (10 and 11) of which the first one (10) acts on said first hooking/releasing device (18, 62) and the second one (11) is of the progressive type and acts in progression on said first hooking/releasing device (18, 62) at first if it has not been released yet and then on said second hooking/releasing device (16, 42), such that said stylet slider (33) and said cannula slider (31) are released through a single control or in respective separate sequence by two separate controls.

2. Appliance according to claim 1, **characterised by** the fact that said stylet slider (33) includes a central body (60), four guiding flaps (56, 57, 58, 59) fitted for engaging two opposite guiding channels (12a, 29a) on opposite inner walls of handpiece (1a, 1b), and elastic bridge (61) carrying a holding tooth (62).

3. Appliance according to claim 1 or 2, **characterised by** the fact that said cannula slider (31) includes a central body (40), four guiding flaps (34, 35, 36, 37) fitted for engaging two opposed guiding channels (15a, 26a) on opposite inner walls of handpiece (1a, 1b), and elastic bridge (41) carrying a holding tooth (42).

4. Appliance according to any of the preceding claims, **characterised by** the fact that said first hooking/releasing device (18, 62) of stylet slider (33) includes a fixed holding tooth (18) carried towards its inner part by box-handpiece (1a) and a mobile holding tooth (62) carried by stylet slider (33) where said mobile tooth (62) engages said fixed tooth (18) to keep stylet slider (33) wound;
- by the fact that said second hooking/releasing device (16, 42) includes a fixed holding tooth (16) carried by handpiece-box (1a) towards its inner part and a mobile holding tooth (42) carried by cannula slider (31) where said mobile tooth (42) engages said fixed tooth (16) to keep cannula slider (31) wound,
- by the fact that said first control push-button (10) if pressed, moves said mobile tooth (62) of stylet slider in order to release it from respective fixed tooth (18),
- by the fact that said second control push-button (11) if pressed moves in progression said mobile tooth (62) of stylet slider (33) first in order to release it from respective fixed tooth (18) if said stylet slider (33) is still in its wound position, and moves in progression said mobile tooth (42) of cannula slider (31) in order to release it from respective fixed tooth (16).

5. Appliance according to claim 4, **characterised by** the fact that said two push-buttons (10, 11) are otained by making two bending portions (10, 11) of a wall (6) of handpiece.

6. Appliance according to any of the preceding claims, **characterised by** the fact that said loading means include a loader slider (32) which is mobile lengthwise guided in said handpiece-box (1a, 1b) fitted for engaging cannula slider (31) and stylet slider (33) to carry them to their respective wound positions and by the fact that said loader slider (32) includes a couple of wings (50a, 50b) projecting towards box-handpiece (1a, 1b) outer part through slots (9, 30) made on it in order to allow the operation of said loader slider (32) from outside.

7. Appliance according to claim 6, **characterised by** the fact that said loader slider (32) includes elastic means (70) facilitating the return stroke after the load stroke.

8. Appliance according to claim 6 or 7, **characterised by** the fact that said loader slider (32) includes a head (48) equipped with a selector device (51a, 51b, 55, 53) which intercepts and drags cannula slider (31) with itself during a first stroke carrying it to the wound position and during a second stroke to drag stylet slider (33) with itself carrying it to the wound position.

9. Appliance according to claim 8, **characterised by** the fact that said loader slider (32) includes a spring guiding rod (49) on which a spring (70) is mounted which is suitable for performing return strokes of said loader slider (32) after winding active strokes of cannula slider (31) and winding strokes of stylet slider (33) and by the fact that said spring guiding rod (49) slides in guided manner lengthwise in said cannula slider (31).

## Patentansprüche

1. Chirurgisches Gerät für die Biopsie mit einer aus einem Stilett (68) und einer koaxial dazu angeordneten Kanüle (80) bestehenden Nadel (8), die sich auf einer Längsachse (y) in axialer Richtung erstreckt und ein freies, in die Entnahmezone einführbares Ende und ein weiteres Ende hat, das in einem kastenförmigen Handgriff (1) untergebracht ist, der zur automatischen Durchführung der Entnahmeoperation Steuereinrichtungen enthält, welche umfassen:
- einen Stilett-Schieber (33), der in dem Handgriff (1) in Längsrichtung geführt ist und das Stilett (68) trägt und in Längsrichtung vorschiebt,
- einen Kanülen-Schieber (31), der in dem Handgriff (1) in Längsrichtung geführt ist und die Kanüle (80) trägt und in Längsrichtung vorschiebt,
- erste elastische Mittel (64), die den Stilett-Schieber (33) in Längsrichtung elastisch belasten,
- zweite elastische Mittel (45), die den Kanülen-Schieber (31) in Längsrichtung elastisch belasten,
- Halte- und Freigabemittel, die den Stilett-Schieber (33) und den Kanülen-Schieber (31) in der belasteten Stellung halten und diese in automatischer schneller Folge oder in getrennter Folge freigeben,
- Mittel zum Überführen des Stilett-Schiebers (33) und des Kanülen-Schiebers (31) in die belastete Stellung,
- wobei der Stilett-Schieber (33) in einer Längsebene verschieblich ist, die seitlich neben der Achse (y) der Nadel (8) liegt, und einen Kopf (66) aufweist, der sich in Richtung auf diese Achse (y) und darüber hinaus erstreckt und so ausgebildet ist, daß er das Stilett (68) koaxial zu der Achse (y) ausrichtet,
- wobei ferner der Kanülen-Schieber (31) in einer Längsebene verschieblich ist, die seitlich neben der Achse (y) der Nadel (8) und außerhalb des Bewegungsbereiches des Stilett-Schiebers (33) liegt, und einen Kopf (38) hat, der sich in Richtung auf die Achse (y) und darüber hinaus erstreckt und so ausgebildet ist, daß er die Kanüle (80) koaxial zu der Achse (y) ausrichtet,
**dadurch gekennzeichnet, daß** die Halte- und Freigabemittel ein unabhängiges erstes Verriegelungs- und Entriegelungsorgan (18, 62) für den Stilett-Schieber (33), ein unabhängiges zweites Verriegelungs- und Entriegelungsorgan (16, 42) für den Kanülen-Schieber (31) und zwei unabhängige, voneinander getrennte Betätigungs-Drucktasten (10 und 11) aufweisen, von denen die erste Taste (10) das erste Verriegelungs- und Entriegelungsorgan (18, 62) betätigt, während die zweite, progressiv wirkende Taste (11) nacheinander zunächst das erste Verriegelungs- und Entriegelungsorgan (18, 62), sofern dieses noch nicht gelöst ist, und danach das zweite Verriegelungs- und Entriegelungsorgan (16, 42) betätigt, derart, daß der Stilett-Schieber (33) und der Kanülen-Schieber (31) mittels eines einzigen Steuerbefehls oder in getrennter Folge durch zwei getrennte Steuerbefehle freigegeben werden.

2. Gerät nach Anspruch 1, **dadurch gekennzeichnet, daß** der Stilett-Schieber (33) einen Zentralkörper (60), vier Führungszungen (56, 57, 58, 59) für den Eingriff in zwei einander gegenüberliegende Führungskanäle (12a, 29a) an gegenüberliegenden Innenwänden des Handgriffs (1a, 1b) und eine elastische Brücke (61) mit einer Haltenase (62) aufweist.

3. Gerät nach Anspruch 1 oder 2, **dadurch gekennzeichnet, daß** der Kanülen-Schieber (31) einen Zentralkörper (40), vier Führungszungen (34, 35, 36, 37) für den Eingriff in zwei einander gegenüberliegende Führungskanäle (15a, 26a) an gegenüberliegenden Innenwänden des Handgriffs (1a, 1b) und eine elastische Brücke (41) mit einer Haltenase (42) aufweist.

4. Gerät nach einem der vorhergehenden Ansprüche, **dadurch gekennzeichnet, daß** das erste Verriegelungs- und Entriegelungsorgan (18, 62) des Stilett-Schiebers (33) eine feste Haltenase (18), die von dem kastenförmigen Handgriff (1a) nach innen absteht, und eine bewegliche Haltenase (62) aufweist, die von dem Stilett-Schieber (33) getragen wird und die in Eingriff mit der festen Haltenase (18) kommt, um den Stilett-Schieber (33) in vorgespannter Stellung zu halten,
- daß das zweite Verriegelungs- und Entriegelungsorgan (16, 42) eine feste Haltenase (16), die von dem kastenförmigen Handgriff (1a) nach innen absteht, und eine bewegliche Haltenase (42) aufweist, die von dem Kanülen-Schieber (31) getragen wird und in Eingriff mit der festen Haltenase (16) kommt, um den Kanülen-Schieber (31) in der gespannten Stellung zu halten,
- daß die erste Betätigungs-Drucktaste (10) in niedergedrückter Stellung die bewegliche Haltenase (62) des Stilett-Schiebers so bewegt, daß diese von der zugehörigen, festen Haltenase (18) freikommt,
- daß die zweite Betätigungs-Drucktaste (11) in niedergedrückter Stellung, nacheinander zunächst die bewegliche Haltenase (62) des Stilett-Schiebers (33) verschiebt, so daß diese von der zugehörigen festen Haltenase (18) freikommt, wenn sich der Stilett-Schieber (33) noch in der gespannten Position befindet, und anschließend die bewegliche Haltenase (62) des Kanülen-Schiebers (31) so verschiebt, daß diese von der zugehörigen festen Haltenase (16) freikommt.

5. Gerät nach Anspruch 4, **dadurch gekennzeichnet, daß** die beiden Betätigungs-Drucktasten (10, 11) durch zwei biegsame Bereiche (10, 11) einer Wand (6) des Handgriffs gebildet sind.

6. Gerät nach einem der vorhergehenden Ansprüche, **dadurch gekennzeichnet, daß** die Mittel zur Belastung einen Spannschieber (32) aufweisen, der in dem kastenförmigen Handgriff (1a, 1b) in. Längsrichtung beweglich geführt und so ausgebildet ist, daß er den Kanülen-Schieber (31) und den Stilett-Schieber (33) erfaßt, um diese in ihre jeweilige Spannstellung zu bringen, und daß der Spannschieber (32) zwei Flügel (50a, 50b) hat, die durch Schlitze (9, 30) in dem kastenförmigen Handgriff (1a, 1b) nach außen vorstehen, so daß der Spannschieber (32) von außen betätigt werden kann.

7. Gerät nach Anspruch 6, **dadurch gekennzeichnet, daß** der Spannschieber (32) elastische Mittel (70) aufweist, welche nach dem Spannhub den Rückkehrhub unterstützen.

8. Gerät nach Anspruch 6 oder 7, **dadurch gekennzeichnet, daß** der Spannschieber (32) einen Kopf (48) aufweist, der mit einem Wählorgan (51a, 51b, 55, 53) ausgerüstet ist, welches während des ersten Hubs den Kanülen-Schieber (31) erfaßt und mitzieht, um diesen in die gespannte Stellung zu bringen, während es bei einem zweiten Hub den Stilett-Schieber (33) erfaßt und mitnimmt, um diesen in die gespannte Stellung zu bringen.

9. Gerät nach Anspruch 8, **dadurch gekennzeichnet, daß** der Spannschieber (32) eine federbelastete Führungsstange (49) aufweist, auf die eine Feder (70) aufgesetzt ist, welche den Rückwärtshub des Spannschiebers (32) nach den aktiven Hüben zum Spannen des Kanülen-Schiebers (31) und des Stilett-Schiebers (33) zurückdrückt, und daß die federbelastete Führungsstange (49) in dem Kanülen-Schieber (31) in Längsrichtung verschieblich geführt ist.

## Revendications

1. Appareil chirurgical pour biopsie comprenant une aiguille (8) composée d'un stylet (68) et d'une canule (80) disposés coaxialement et s'étendant axialement le long d'un axe longitudinal (Y), ayant une extrémité libre destinée à être insérée dans la zone de prélèvement et l'autre extrémité placée dans une pièce de préhension en forme de boîte (1) contenant des moyens de commande pour automatiser les opérations de prélèvement, lesdits moyens comprenant :
- un coulisseau de stylet (33) guidé à coulissement longitudinal à l'intérieur de ladite pièce de préhension (1) pour porter et avancer le stylet (68) longitudinalement ;
- un coulisseau de canule (31) guidé à coulissement longitudinal à l'intérieur de ladite pièce de préhension (1) pour supporter et avancer la canule (80) longitudinalement ;
- un premier moyen élastique (64) adapté pour charger élastiquement ledit coulisseau de stylet (33) longitudinalement ;
- un second moyen élastique (45) adapté pour charger élastiquement ledit coulisseau de canule (31) longitudinalement ;
- des moyens de retenue et de libération pour retenir ledit coulisseau de stylet (33) et ledit coulisseau de canule (31) dans la position chargée et pour les libérer dans une séquence automatique rapide respective ou dans une séquence séparée ;
- des moyens pour porter ledit coulisseau de stylet (33) et ledit coulisseau de canule (31) dans la position chargée ;
- ledit coulisseau de stylet (33) coulissant dans un plan longitudinal disposé latéralement par rapport à l'axe (Y) de l'aiguille (8) et comprenant une tête (66) s'étendant vers et au-delà dudit axe (Y) et capable d'amener le stylet (68) coaxialement par rapport audit axe (Y) ;
- ledit coulisseau de canule (31) coulissant dans un plan longitudinal disposé latéralement par rapport à l'axe (Y) de l'aiguille (8) et n'interférant pas dans la plage de fonctionnement du coulisseau de stylet (33) et comprenant une tête (38) s'étendant vers et au-delà dudit axe (Y) et capable d'amener la canule (80) coaxialement par rapport audit axe (Y) ;
- **caractérisé par le fait que** lesdits moyens de retenue et de libération comprennent un premier dispositif d'accrochage et de relâchement indépendant (18, 62) pour le coulisseau de stylet (33), un second dispositif d'accrochage et de relâchement indépendant (16, 42) pour le coulisseau de canule (31), et deux boutons poussoirs de commande (10 et 11) séparés indépendants dont le premier (10) agit sur ledit premier dispositif d'accrochage et de relâchement (18, 62) et le second (11) est de type progressif et agit progressivement sur ledit premier dispositif d'accrochage et de relâchement (18, 62) s'il n'a pas encore été tout d'abord relâché puis sur ledit second dispositif d'accrochage et de relâchement (16, 42), de telle sorte que ledit coulisseau de stylet (33) et ledit coulisseau de canule (31) sont libérés au moyen d'une commande unique ou dans une séquence séparée respective par deux commandes séparées.

2. Dispositif selon la revendication 1, **caractérisé par le fait que** ledit coulisseau de stylet (33) comprend un corps central (60), quatre flasques de guidage (56, 57, 58, 59) adaptés pour s'engager dans deux canaux de guidage opposés (12a, 29a) sur des parois intérieures opposées de la pièce de préhension (1a, 1b), et un pont élastique (61) portant une dent de retenue (62).

3. Dispositif selon la revendication 1 ou la revendication 2, **caractérisé par le fait que** ledit coulisseau de canule (31) comprend un corps central (40), quatre flasques de guidage (34, 35, 36, 37) adaptés pour s'engager sur deux canaux de guidage opposés (15a, 26a) sur des parois intérieures opposées de la pièce de préhension (1a, 1b), et un pont élastique (41) portant une dent de retenue (42).

4. Dispositif selon l'une quelconque des revendications précédentes, **caractérisé par le fait que** ledit premier dispositif d'accrochage et de relâchement (18, 62) de coulisseau de stylet (33) comprend une dent de retenue fixe (18) portée en direction de sa partie intérieure par la pièce de préhension en forme de boîte (1a) et une dent de retenue mobile (62) portée par le coulisseau de stylet (33) de façon que ladite dent mobile (62) s'engage sur ladite dent fixe (18) pour entourer et maintenir le coulisseau de stylet (33) ;
- **par le fait que** ledit second dispositif d'accrochage et de relâchement (16, 42) comprend une dent de maintien fixe (16) portée par la pièce de préhension en forme de boîte (1a) en direction de sa partie intérieure et une dent de retenue mobile (42) portée par le coulisseau de canule (31) de façon que ladite dent mobile (42) s'engage sur ladite dent fixe (16) pour entourer et maintenir le coulisseau de canule (31),
- **par le fait que** ledit premier bouton poussoir de commande (10), s'il est pressé, déplace ladite dent mobile (62) de coulisseau de stylet de façon à l'éloigner de la dent fixe respective (18),
- **par le fait que** ledit second bouton poussoir de commande (11), s'il est pressé, déplace progressivement ladite dent mobile (62) de coulisseau de stylet (33) tout d'abord pour l'éloigner de ladite dent fixe respective (18) si ledit coulisseau de stylet (33) est encore dans sa position entourée, et déplace progressivement ladite dent mobile (42) de coulisseau de canule (31) pour l'éloigner de la dent fixe respective (16).

5. Dispositif selon la revendication 4, **caractérisé par le fait que** lesdits deux boutons poussoirs (10, 11) sont obtenus en réalisant deux portions recourbées (10, 11) d'une paroi (6) de la pièce de préhension.

6. Dispositif selon l'une quelconque des revendications précédentes, **caractérisé par le fait que** ledit moyen de charge comprend un coulisseau de charge (32) qui est guidé en déplacement longitudinal dans ladite pièce de préhension en forme de boîte (1a, 1b) et qui est adapté pour s'engager sur le coulisseau de canule (31) et sur le coulisseau de stylet (33) pour les porter vers leurs positions respectives d'entourage et **par le fait que** ledit coulisseau de charge (32) comprend deux ailes (50a, 50b) qui dépassent vers l'extérieur de la pièce de préhension en forme de boîte (1a, 1b) à travers des fentes (9, 30) réalisées dans celle-ci pour permettre l'actionnement dudit coulisseau de charge (32) depuis l'extérieur.

7. Dispositif selon la revendication 6, **caractérisé par le fait que** ledit coulisseau de charge (32) comprend un moyen élastique (70) qui facilite la course de retour après la course de charge.

8. Dispositif selon la revendication 6 ou la revendication 7, **caractérisé par le fait que** ledit coulisseau de charge (32) comprend une tête (48) équipée avec un dispositif sélecteur (51a, 51b, 55, 53) qui intercepte et tire le coulisseau de canule (31) avec lui-même pendant une première course le déplaçant jusqu'à la position d'entourage et pendant une seconde course pour tirer le coulisseau de stylet (33) avec lui-même en le déplaçant jusqu'à la position d'entourage.

9. Dispositif selon la revendication 8, **caractérisé par le fait que** ledit coulisseau de charge (32) comprend une tige de guidage de ressort (49) sur laquelle un ressort (70) est monté qui est approprié pour réaliser les courses de retour dudit coulisseau de charge (32) après les courses actives d'entourage du coulisseau de canule (31) et les courses d'entourage du coulisseau de stylet (33) et **par le fait que** ladite tige de guidage de ressort (49) coulisse en étant guidée longitudinalement dans ledit coulisseau de canule (31).
